# EUROPEAN PATENT SPECIFICATION

(11) **EP 2 661 492 B1**
(45) Date of publication and mention of the grant of the patent: **04.10.2017**
(21) Application number: 12731900.2
(22) Date of filing: 06.01.2012
(51) Int. Cl.: C12N 5/10, C12N 15/12, C12N 15/52, C07K 9/00, A61K 8/60, C12N 9/10

(54) **METHOD OF PRODUCTION OF RECOMBINANT GLYCOPROTEINS WITH INCREASED CIRCULATORY HALF-LIFE IN MAMMALIAN CELLS**
VERFAHREN ZUR PRODUKTION REKOMBINANTER GLYCOPROTEINE MIT ERHÖHTER KREISLAUF-HALBWERTSZEIT IN SÄUGETIERZELLEN
PROCÉDÉ DE PRODUCTION DE GLYCOPROTÉINES RECOMBINÉES PRÉSENTANT UNE DEMI-VIE CIRCULANTE ACCRUE DANS DES CELLULES DE MAMMIFÈRE

(30) Priority: 06.01.2011 US 201161430256 P
(43) Date of publication of application: 13.11.2013
(73) Proprietor: The Johns Hopkins University, Baltimore, MD 21218 (US)
(72) Inventor: BETENBAUGH, Michael, J., Baltimore, MD 21239 (US)
(74) Representative: Cornish, Kristina Victoria Joy
(86) International application number: PCT/US2012/020535
(87) International publication number: WO 2012/094627

(56) References cited:
- WO-A2-03/061562
- US-A1- 2002 045 207
- US-A1- 2005 123 564
- MONACO L ET AL: "GENETIC ENGINEERING OF ALPHA2,6-SIALYLTRANSFERASE IN RECOMBINANT CHO CELLS AND ITS EFFECTS ON THE SIALYLATION OF RECOMBINANT INTERFERON-GAMMA", 1 January 1996 (1996-01-01), CYTOTECHNOLOGY, KLUWER ACADEMIC PUBLISHERS, DORDRECHT, NL, PAGE(S) 197 - 203, XP009064109, ISSN: 0920-9069 * abstract * * page 200, left-hand column, paragraph 2 - right-hand column, paragraph 2 *
- PRATI E G P ET AL: "ENGINEERING OF COORDINATED UP- AND DOWN-REGULATION OF TWO GLYCOSYLTRANSFERASES OF THE O-GLYCOSYLATION PATHWAY IN CHINESE HAMSTER OVARY (CHO) CELLS", 5 September 2002 (2002-09-05), BIOTECHNOLOGY AND BIOENGINEERING, WILEY & SONS, HOBOKEN, NJ, US, PAGE(S) 580 - 585, XP008062016, ISSN: 0006-3592 * page 243, left-hand column, paragraph 2 - right-hand column, paragraph 3 *
- BRAGONZI ET AL.: 'A new chinese hamster ovary cell line expressing alpha2,6- sialyltransferase used as universal host for the production of human-like sialylated recombinant glycoproteins' BIOCHEMICA ET BIOPHYSICA ACTA vol. 1474, 2000, pages 273 - 282, XP004276566
- KOLARICH ET AL.: 'Glycoproteomic characterization of butyrylcholinesterase from human plasma' PROTEOMICS vol. 8, 2008, pages 254 - 263, XP055070002
- CHITLARU ET AL.: 'Overloading and removal of N-glycosylation targets on human acetylcholinesterase: effects on glycan composition and circulatory residence time' BIOCHEMICAL JOURNAL vol. 262, 2002, pages 619 - 631, XP055113019
- CHITLARU ET AL.: 'Modulation of circulatory residence of recombinant acetylcholinesterase through biochemical or genetic manipulation of sialylation levels' BIOCHEMICAL JOURNAL vol. 336, 1998, pages 647 - 658, XP055113021
- NGANTUNG ET AL.: 'RNA interference of sialidase improves glycoprotein sialic acid content consistency' BIOTECHNOLOGY AND BIOENGINEERING vol. 95, no. 1, 05 September 2006, pages 107 - 119, XP002416179

## Description

### BACKGROUND OF THE INVENTION

### FIELD OF THE INVENTION

The invention relates generally to recombinant expression systems and more specifically to methods for the biosynthesis of glycoproteins with increased alpha 2-6 sialic acid content.

### BACKGROUND INFORMATION

The capacity of organophosphorus (OP) agents to inhibit acetylcholinesterase (AChE) in nerve cells has led to their use as insecticides, herbicides and potent nerve agents including Sarin, Tabun, Soman and VX. Their neurotoxic effect on the cholinergic nervous system can lead to a choking sensation, loss of vision, excessive salivation, stomach cramps, vomiting, diarrhea, muscle spasms, unconsciousness and death if not treated properly. The cause of the reaction is the loss of the capacity to break down acetylcholine, thereby leading to overstimulation of the nerve cells.

Because exposure to OP represents a potential chemical danger in the future, agents that can be used for prophylaxis against these chemicals represent a significant opportunity to protect warfighters and civilians from chemical poisoning. One means to protect soldiers against OP agents is through injection of bioscavengers that bind to these OP agents and prevent them from reaching the site of action.

OP nerve agents such sarin, soman, and VX represent some of the most dangerous chemical weapons threats our warfighters and civilian population face as these lethal agents can be produced by a foreign entity or terrorist organization. As a result, these agents may come into the possession of organizations counter to American interests. Furthermore, these agents have the potential to incapacitate, harm or even kill thousands of warfighters or civilians if the agents are spread through a large area. As a result, medical countermeasures to prevent their toxicity would be extremely helpful in preventing or mitigating the toxic effects manifested by these OP nerve agents. OP-scavengers, when used as a protective prophylactic, currently represent one of the best alternatives to prevent or minimize the harm to warfighters and civilians caused by exposure to these neurotoxins in a chemical danger zone. The most likely end-users for OP-scavengers are American and allied warfighters in a zone of warfare in which there is significant opportunity or danger for exposure to such chemical nerve agents. A second end-user would be civilians under American protection who are under significant risk of chemical attack by terrorists or rogue regimes.

One of the most prominent bioscavenger candidates is butyrylcholinesterase (BChE), including human plasma butyrylcholinesterase (huBChE). BChE is a natural plasma enzyme of cholineesterase family found in humans and other animals. BChE is a tetrameric serine esterase with a molecular mass of approximately 340 kDa and a sustained half-life in the body. While the exact physiological function of huBChE is not yet known, the enzyme can prevent intoxication of animals exposed to OP compounds. In its role as a bioscavenger, huBChE binds directly to the nerve agents, sequestering it from acting on the nerve agent's principal target of acetylcholinesterase. In relation to other enzymatic scavengers of OP compounds, huBChE has a broad spectrum of activity and limited, if any, physiological side effects. Moreover, human BChE (huBChE) derived from plasma has a sustained and long mean residence time in the body in the range of tens of hours. Thus, administration of exogenous huBChE represents a potentially invaluable strategy for the prevention of OP agent toxicity to exposed individuals. However, this bioscavenger does not degrade the nerve agents and, as a result, doses on the order of mg/kg of body mass are needed for the protein scavenger to be effective.

huBChE can be obtained from human plasma, however, this approach is not optimal as it is difficult to obtain the large amounts that would be needed in a possible military emergency, particularly in view of the importance of plasma for other unmet medical needs. Because huBChE is effective for protection in a 1:1 stoichiometry of protein to OP agent, alternative sources of the enzyme in amounts sufficient to be useful for the military are required. Consequently, the production of large quantities of effective huBChE presents a major obstacle for the military for successful prophylaxis against exposure of warfighters to OP neurotoxins.

Recombinant expression systems for the large-scale production of rhuBChE have been explored and, as a result, the protein has been expressed in expression systems including E. coli, mammals, and transgenic animals. Given the success of CHO-derived products in the biotechnology industry, it was speculated that rhuBChE from CHO, and perhaps other mammalian cells, would also be effective as a replacement for human plasma-derived BChE. Unfortunately in all cases, recombinant human BChE (rhuBChE) has not been as effective as plasma-derived huBChE, predominantly because the circulatory half-life can be many fold shorter, resulting in an agent that does not work for long periods in the field. The reason that current rhuBChE is not effective is ascribed to the expression systems used to produce this protein, which do not have the synthetic capability necessary for generating a product with similar properties as the native huBChE. Since warfighters may have extended periods of potential exposure, it is desirable to develop a BChE bioscavenger with an extended circulatory half-life in the body.

Recently, researchers have attempted to solve the problem of limited half-life of the recombinant human form of BChE (rhuBChE) by attaching polyethylene glycol (PEG) molecules to increase the size and perhaps reduce the immunogenicity of the protein. However, this modification has limitations as well. In one study, recombinant human BChE exhibited a mean residence time 2.5 fold shorter than the mean residence time of native serum-derived HuBChE in mice. In order to improve the half-life, researchers chemically modified the recombinant HuBChE (rHuBChE) by addition of PEG molecules. The addition of PEG indeed increased the mean residence time to 36.2 hours; however, this was still less than the value of the plasma derived form. In another study, the PEGylation was undertaken with native and recombinant Macaque BChE (MaBChE) and the pharmacokinetic profile represented by the area under the curve (AUC) also improved but was still less than the native MaBChE. Furthermore, repeated injections of PEG-rhuBChE produced several fold higher anti-rhuBChE antibodies in mice than the unconjugated enzymes. While in some cases reduced immunogenicity has been observed following PEGylation of enzymes, cytokines and hormones, administration of PEGylated interferon-β-1a in monkeys actually resulted in increased immunogenicity.

The current state of the art limits the availability of BChE exclusively to natural sources of human plasma. This drastically decreases the potential number of warfighters and civilians who may be afforded protection and doses available as there is currently only a limited supply of plasma. This is especially problematic as chemical agents can be used as a weapon spread over large population regions. Moreover, current supplies of rhuBChE have been unsatisfactory in terms of circulatory residence time because the expression systems used were incapable of generating a product which had the same properties and biological effectiveness as the natural, plasma-derived product.

### SUMMARY OF THE INVENTION

### The invention is set out in the claims.

The present invention is based on the seminal discovery that the *in vivo* circulatory half-life of glycoproteins is modulated by the sialic acid content and nature of the carbohydrate linkage. Sialic acid attachments on glycoproteins, such as butyrlcholinesterase (BChE), are critical for extended circulatory lifetime. Alpha2-3 sialic acid linkages may be more susceptible to enzymatic degradation than alpha2-6 sialic acid linkages. Post-translational processing events that affect the quaternary structure of glycoproteins also contributes to the pharmacokinetic profile and assembly of glycoproteins into multimers increases their *in vivo* residence time.

The first aspect of the invention is an isolated mammalian cell, such as a Chinese Hamster Ovary (CHO) cell, which includes a heterologous alpha2-6 sialyltransferase (ST6GAL1) nucleic acid sequence optionally wherein the cell is a CHO cell; further comprising a nucleic acid sequence that decreases expression of or silences an alpha2-3sialyltransferase gene; or further comprising means for knock-out of the alpha2-3sialyltransferase gene. The description describes the cell may contain a nucleic acid sequence that encodes for human butyrlcholinesterase (huBChE). In another aspect, the cell includes a nucleic acid sequence that decreases expression of or silences alpha2-3sialyltransferase gene (St3gal1). By way of example, the nucleic acid sequence that silences the St3gal1 gene may be small interfering RNA (siRNA), short interfering RNA, or silencing RNA involved in the RNA intereference (RNAi) pathway. The nucleic acid sequence that silences St3gal1 gene may also be a microRNA (miRNA) molecule. Alternatively, the St3gal1 gene may be knocked-out, for example by zinc finger nucleases. The description describes how the nucleic acid sequences that encode for ST6GAL1 and huBChE, and the nucleic acid sequences that decrease expression of or silence St3gal1 may be simultaneously co-expressed. The description describes how the cell may further include a nucleic acid sequence encoding for an enzyme that reduces or inhibits alpha2-6 sialic acid degradation. The isolated mammalian cell may further include a nucleic acid sequence encoding for the proline-rich attachment domain (PRAD) of the ColQ gene, which may be co-expressed with the nucleic acid sequences that encode for ST6GAL1 and huBChE, and the nucleic acid sequence that silences St3gal1.

Described herein is a recombinant glycoprotein, for example recombinant huBChE (rhuBChE), that contains alpha2-6 sialic acid linkages. The glycoprotein may be a monomer or in a multimeric assembly state, such as a dimer or tetramer. The recombinant glycoproteins provided herein may then have an extended circulatory half-live or mean residence time (MRT).

A method for the biosynthesis of an alpha2-6-rich glycoprotein is provided herein. The method includes culturing an isolated mammalian cell as described in the first aspect, such as a Chinese Hamster Ovary (CHO) cell, containing a heterologous alpha2-6 sialyltransferase (ST6GAL1) nucleic acid sequence under conditions to co-express a nucleic acid sequence that encodes a peptide or protein further comprising inhibiting expression of alpha2-3 sialyltransferase. By way of example, the peptides include, but are not limited to, biological protective agents such as organophosphorus (OP) scavengers. The OP scavenger peptide may be rhuBChE. The description describes how the method further includes modifying the cell to co-express tetramer assembly chaperones, such as PRAD, thereby generating glycoprotein tetramers.

According to the method provided herein, the alpha2-6 content in a glycoprotein may be increased, for example, by reducing or inhibiting degradation of alpha2-6 sialic acid; by increasing the number of and length of N-glycan branches; by increasing the rate of N-glycan branching; or by increasing the CMP-sialic acid content or pool, thereby providing a glycoprotein that is rich in alpha2-6 sialic acid linkages.

The description describes how alpha2-6 sialic acid degradation is reduced or inhibited by increasing activity of an enzyme that prevents alpha2-6 sialic acid degradation, such as a fucosyltransferase enzyme. Examples of fucosyltransferase enzymes include, but are not limited to alpha3fucosyltransferase (alpha3FucT), alpha3,4 fucosyltransferase (FucTLe) or alpha2fucosyltransferase (FucTLe). The enzyme activity may be increased in an enzyme that prevents, inhibits or decreases alpha2-6 sialic acid degradation by expressing the gene encoding for the enzyme. By way of example, the activity of a fucosyltransferase may be increased by increasing expression of FUT1, FUT2, FUT3, FUT4, FUT5, FUT6, FUT7, FUT8, or FUT9.

The description describes how alpha2-6 sialic acid degradation is reduced or inhibited by decreasing activity of an enzyme that promotes alpha2-6 sialic acid degradation including, but not limited to, sialidase or neuramidase enzymes.

The description describes how by increasing the number or the length of N-glycan branches, according to the method of the disclosure, the alpha2-6 content in a glycoprotein may be increased. The number of N-glycan branches may be increased by increasing activity of galactose transferases or GIcNAc-transferases, such as β6-GIcNAc-transferase (IGnT). The length of the N-glycan branches may be increased by, for example, increasing the number of polylactosamines. The number of polylactosamines may be increased by increasing expression of beta3-GlcNAC transferase (iGnT).

The description describes a method for producing rhuBChE. The method includes culturing a mammalian cell that co-expresses huBChE, alpha2-6 sialyltransferase, and PRAD in cell culture medium, thereby producing rhuBChE. The rhuBChE may be isolated from the culture medium. One example of a suitable culture medium is a serum-free medium.

### BRIEF DESCRIPTION OF THE DRAWINGS

Figure 1 is a schematic representation showing the quaternary structure of butyrylcholinesterase (BChE). 1A. depicts the BChE monomer; 1B-D. depict the BChE in the tetrameric assembly state.
Figure 2 is a diagram that shows the chemical structure of sialic acid. 1A. depicts the alpha2-3 sialic acid linkage and 1B. shows the alpha2-6 sialic acid linkage.
Figure 3 is a simplified schematic representation of the sialylation mammalian pathway that indicates the synthesis of sugar nucleotide CMP-Neu5Ac (CMP-sialic acid) and sialylation of BChE.
Figure 4 are graphic representations of the circulatory time of wild-type (4A) and recombinant BChE (4B) and the circulatory time after removal of sialic acid in native huBCHE (4C-D).

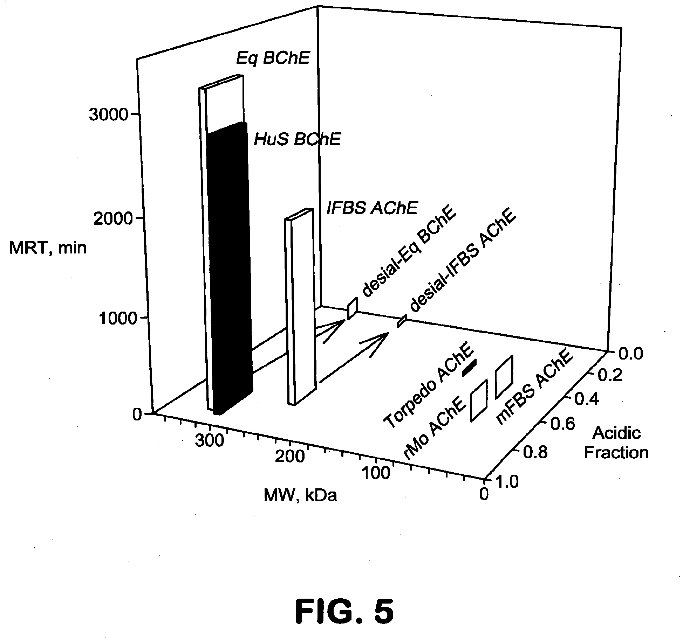

Figure 5 is a three-dimensional plot of mean residence time (MRT) on a molecular weight/percent acidic fraction grid.

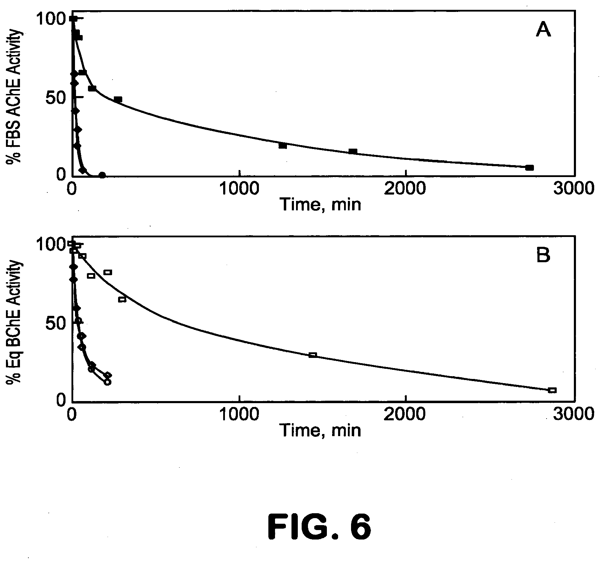

Figure 6 is a graphic representation of the time course of FBS AChE and Eq BChE in the circulation of mice. Stability of FBS AChE (A) and Eq (B) in the circulation of mice following an i.v. injection of 50-80 units if ChE/animal are shown. Curves depict the time course of ChEs in individual mice and were generated in accordance with monoexponential or biexponential decay equations. Each data point is an average of two measurements. Symbols: squares represent native and others represent deglycosylated and desialylated ChEs.

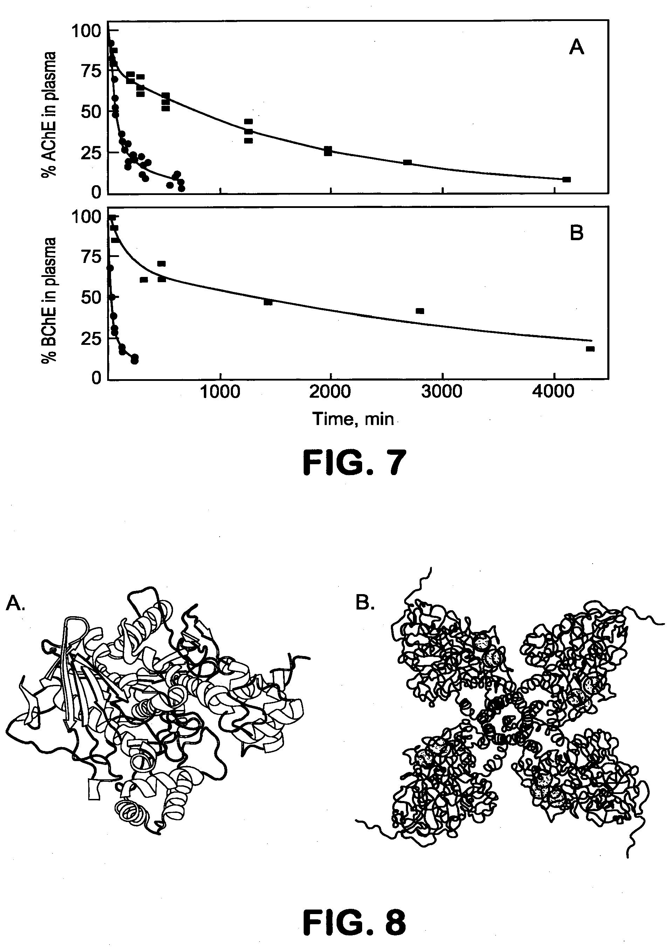

Figure 7 is a graphic representation of the time course of ChEs in the circulation of mice. Individual time courses of ChEs following their intravenous injection into the tail vein of Balb/c mice are shown (A) where tFBS AChE solid circle symbol represents 100 units/animal (three experiments) and mFBS AChE hollow triangle represents 100 units/animal (six experiments). HuS BChE (B) where the inverted hollow triangle symbol represents 39 units/animal (two experiments) and rHuBChE where the solid diamond symbol represents 60 units per animal (four experiments). Curve fitting was carried out in accordance with the equation. Percent ChE activity was calculated by dividing the plasma activity at time = *t* by the activity at *t* = 0 (obtained by extrapolating the curve to *t* =0)*.*
Figure 8 is a schematic representation of the monomeric (A) and tetrameric (B) forms of BChE.

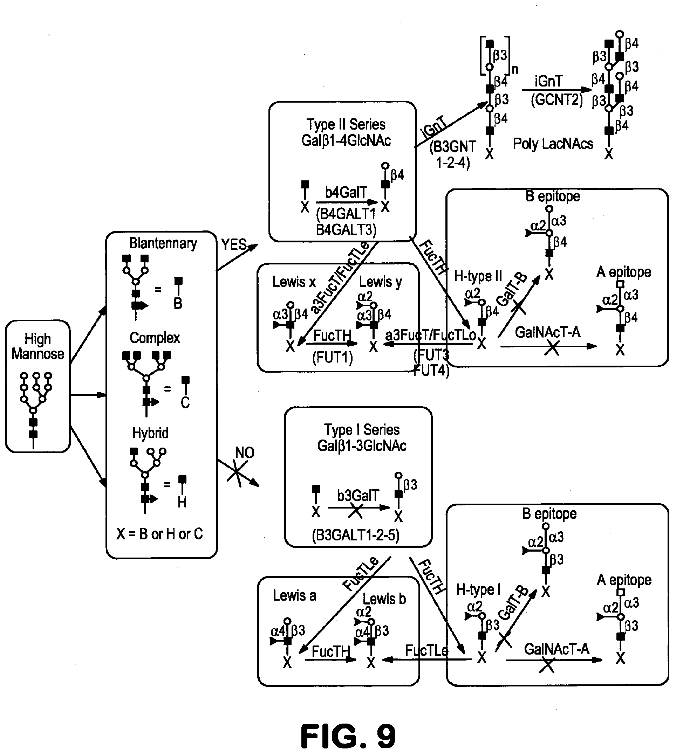

Figure 9 is a schematic representation of a modified N-glycosylation pathway characteristic of high and low passage mammalian cell lines The steps to elaborate the glycan structures corresponding to both LNCaP cell lines are represented in a simplified N-glycosylation pathway according to transcription expression data as well as the mass spectra structural data. When indicated, genes in the pathway are indicated in parenthesis and located below their corresponding enzymes. The "Xs" in the pathway are indicative of the absence or low level of the corresponding enzymes. Initial steps of glycan formation as well as sialylation are omitted.

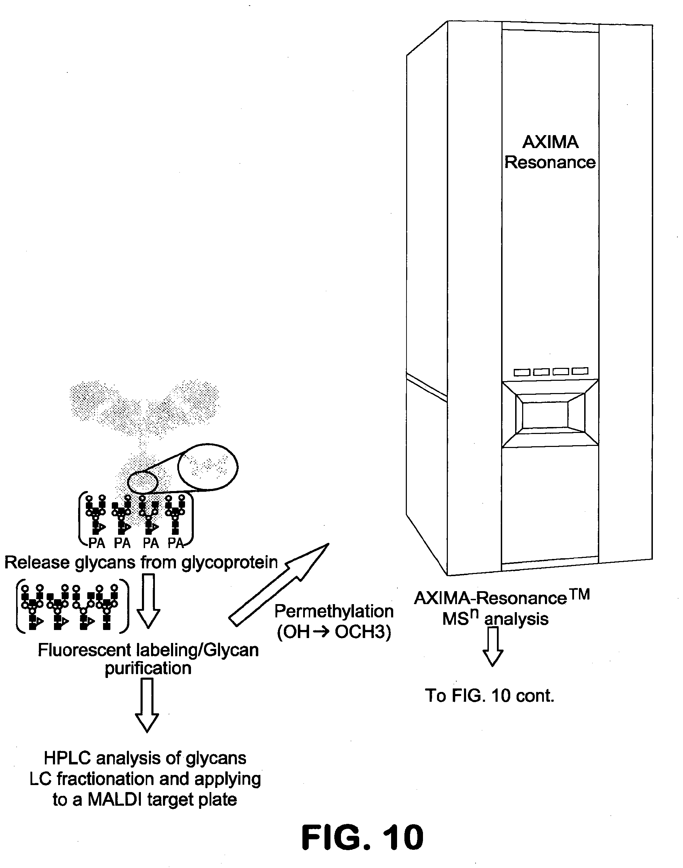

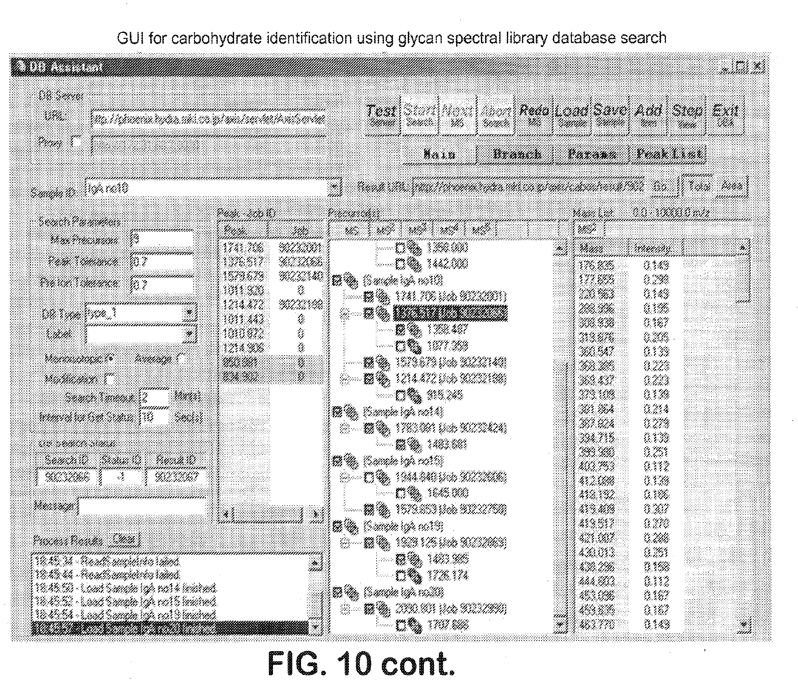

Figure 10 is a flow-chart representation of glycan preparation and analysis using HPLC and LC-MS/MSn technology.

### DETAILED DESCRIPTION OF THE INVENTION

Unless otherwise defined herein, scientific and technical terms used in connection with the present invention shall have the meanings that are commonly understood by those of ordinary skill in the art. Further, unless otherwise required by context, singular terms shall include pluralities and plural terms shall include the singular. The methods and techniques of the present disclosure are generally performed according to conventional methods well known in the art. Generally, nomenclatures used in connection with, and techniques of biochemistry, enzymology, molecular, and cellular biology, microbiology, genetics and protein and nucleic acid chemistry and hybridization described herein are those well-known and commonly used in the art.

The methods and techniques of the present invention are generally performed according to conventional methods well-known in the art and as described in various general and more specific references that are cited and discussed throughout the present specification unless otherwise indicated. See, e.g., Sambrook et al. Molecular Cloning: A Laboratory Manual, 2d ed., Cold Spring Harbor Laboratory Press, Cold Spring Harbor, N.Y. (1989); Ausubel et al., Current Protocols in Molecular Biology, Greene Publishing Associates (1992, and Supplements to 2002); Harlow and Lane Antibodies: A Laboratory Manual Cold Spring Harbor Laboratory Press, Cold Spring Harbor, N.Y. (1990); Introduction to Glycobiology, Maureen E. Taylor, Kurt Drickamer, Oxford Univ. Press (2003); Worthington Enzyme Manual, Worthington Biochemical Corp. Freehold, N.J.; Handbook of Biochemistry: Section A Proteins Vol I 1976 CRC Press; Handbook of Biochemistry: Section A Proteins Vol II 1976 CRC Press; Essentials of Glycobiology, Cold Spring Harbor Laboratory Press (1999). The nomenclatures used in connection with, and the laboratory procedures and techniques of, molecular and cellular biology, protein biochemistry, enzymology and medicinal and pharmaceutical chemistry described herein are those well known and commonly used in the art.

The following terms, unless otherwise indicated, shall be understood to have the following meanings:
As used herein, the term "N-glycan" refers to an N-linked oligosaccharide, e.g., one that is attached by an asparagine N- acetylglucosamine linkage to an asparagine residue of a polypeptide. N -glycans have a common pentasaccharide core of Man3GlcNAc2 ("Man" refers to mannose; "Glc" refers to glucose; and "NAc" refers to N-acetyl; GlcNAc refers to N-acetylglucosamine). The term "trimannose core" used with respect to the N-glycan also refers to the structure Man3GlcNAc2 ("Man3 "). N-glycans differ with respect to the number of branches (antennae) comprising peripheral sugars (e.g., fucose and sialic acid) that are added to the Man3 core structure. N-glycans are classified according to their branched constituents (e.g., high mannose, complex or hybrid).

A "high mannose" type N-glycan has five or more mannose residues. A "complex" type N-glycan typically has at least one GlcNAc attached to the 1,3 mannose arm and at least one GlcNAc attached to the 1,6 mannose arm of the trimannose core. Complex N-glycans may also have galactose ("Gal") residues that are optionally modified with sialic acid or derivatives ("NeuAc", where "Neu" refers to neuraminic acid and "Ac" refers to acetyl). A complex N-glycan typically has at least one branch that terminates in an oligosaccharide such as, for example: NeuNAc-; NeuAca2-6GaINAcal-; NeuAca2-3Galbl-3GaINAcal-; NeuAca2-3/6Galbl-4GlcNAcbl-; GlcNAcal-4Galbl-(mucins only); Fucal-2Galbl-(blood group H). Sulfate esters can occur on galactose, GaINAc, and GlcNAc residues, and phosphate esters can occur on mannose residues. NeuAc (Neu: neuraminic acid; Ac:acetyl) can be O-acetylated or replaced by NeuGl (N-glycolylneuraminic acid). Complex N-glycans may also have intrachain substitutions comprising "bisecting" GlcNAc and core fucose ("Fuc"). A "hybrid" N -glycan has at least one GlcNAc on the terminal of the 1,3 mannose arm of the trimannose core and zero or more mannoses on the 1,6 mannose arm of the trimannose core.

Abbreviations used herein are of common usage in the art, see, e.g., abbreviations of sugars, above. Other common abbreviations include "PNGase", which refers to peptide N-glycosidase F (EC 3.2.2.18); "GlcNAc Tr" or "GnT," which refers to N-acetylglucosaminyl Transferase enzymes; *"NANN'* refers to N-acetylneuraminic acid.

The term "enzyme", when used herein in connection with altering host cell glycosylation, refers to a molecule having at least one enzymatic activity, and includes full-lengthenzymes, catalytically active fragments, chimerics, complexes, and the like. A "catalytically active fragment" of an enzyme refers to a polypeptide having a detectable level of functional (enzymatic) activity.

The term "polynucleotide" or "nucleic acid molecule" refers to a polymeric fonn of nucleotides of at least 10 bases in length. The term includes DNA molecules (e.g., cDNA or genomic or synthetic DNA) and RNA molecules (e.g., mRNA or synthetic RNA), as well as analogs of DNA or RNA containing non-natural nucleotide analogs, non-native internucleoside bonds, or both. The nucleic acid can be in any topological conformation. For instance, the nucleic acid can be single-stranded, double-stranded, triple-stranded, quadruplexed, partially double-stranded, branched, hairpinned, circular, or in a padlocked conformation. The term includes single and double stranded forms of DNA. A nucleic acid molecule of this invention may include both sense and antisense strands of RNA, cDNA, genomic DNA, and synthetic forms and mixed polymers of the above. They may be modified chemically or biochemically or may contain non-natural or derivatized nucleotide bases, as will be readily appreciated by those of skill in the art. Such modifications include, for example, labels, methylation, substitution of one or more of the naturally occurring nucleotides with an analog, internucleotide modifications such as uncharged linkages (e.g., methyl phosphonates, phosphotriesters, phosphoramidates, carbamates, etc.), charged linkages (e.g., phosphorothioates, phosphorodithioates, etc.), pendent moieties (e.g., polypeptides), intercalators (e.g., acridine, psoralen, etc.), chelators, alkylators, and modified linkages (e.g., alpha anomeric nucleic acids, etc.) Also included are synthetic molecules that mimic polynucleotides in their ability to bind to a designated sequence via hydrogen bonding and other chemical interactions. Such molecules are known in the art and include, for example, those in which peptide linkages substitute for phosphate linkages in the backbone of the molecule. The nucleic acids (also referred to as polynucleotides) of this invention may include both sense and antisense strands of RNA, cDNA, genomic DNA, and synthetic forms and mixed polymers of the above. They may be modified chemically or biochemically or may contain non-natural or derivatized nucleotide bases, as will be readily appreciated by those of skill in the art. Such modifications include, for example, labels, methylation, substitution of one or more of the naturally occurring nucleotides with an analog, internucleotide modifications such as uncharged linkages (e.g., methyl phosphonates, phosphotriesters, phosphoramidates, carbamates, etc.), charged linkages (e.g., phosphorothioates, phosphorodithioates, etc.), pendent moieties (e.g., polypeptides), intercalators (e.g., acridine, psoralen, etc.), chelators, alkylators, and modified linkages (e.g., alpha anomeric nucleic acids, etc.). Also included are synthetic molecules that mimic polynucleotides in their ability to bind to a designated sequence via hydrogen bonding and other chemical interactions. Such molecules are known in the art and include, for example, those in which peptide linkages substitute for phosphate linkages in the backbone of the molecule.

The term "recombinant host cell" (or simply "host cell"), as used herein, is intended to refer to a cell that has been genetically engineered. A recombinant host cell includes a cell into which a recombinant vector has been introduced. It should be understood that such terms are intended to refer not only to the particular subject cell but to the progeny of such a cell. Because certain modifications may occur in succeeding generations due to either mutation or environmental influences, such progeny may not, in fact, be identical to the parent cell, but are still included within the scope of the term "host cell" as used herein. A recombinant host cell may be an isolated cell or cell line grown in culture or may be a cell which resides in a living tissue or organism. The term "host" refers to any organism, animal or plant, comprising one or more "host cells", or to the source of the "host cells".

The term "peptide" as used herein refers to a short polypeptide, e.g., one that is typically less than about 50 amino acids long and more typically less than about 30 amino acids long. The term as used herein encompasses analogs and mimetics that mimic structural and thus biological function.

The term "polypeptide" as used herein encompasses both naturally-occurring and non-naturally-occurring proteins, and fragments, mutants, derivatives and analogs thereof. A polypeptide may be monomeric or polymeric. Further, a polypeptide may comprise a number of different domains each of which has one or more distinct activities.

"Operatively linked" expression control sequences refers to a linkage in which the expression control sequence is contiguous with the gene of interest to control the gene of interest, as well as expression control sequences that act in trans or at a distance to control the gene of interest.

As used herein, the term "molecule" means any compound, including, but not limited to, a small molecule, peptide, protein, sugar, nucleotide, nucleic acid, lipid, etc., and such a compound can be natural or synthetic.

As used herein, a "CMP-Sia pool" refers to a detectable level of cellular CMP-Sia. The CMP-Sia pool may be the result of the production of CMP-Sia by the host cell, or of the uptake of CMP-Sia from the culture media.

The substrate UDP-GlcNAc is the abbreviation for UDPN- acetylglucosamine. The intermediate ManNAc is the abbreviation for N-acetylmannosamine. The intermediate ManNAc-6-P is the abbreviation for N-acetylmaunosamine- 6-phosphate. The intermediate Sia-9-P is the abbreviation for sialate-9-phosphate. The intermediate Cytidine monophosphate-sialic acid is abbreviated as "CMP-Sia." Sialic acid is abbreviated as "Sia," "Neu5Ac," "NeuAc" or "NANA" herein.

As used herein, the term "sialic acid" refers to a group of molecules where the common molecule includes N -acetyl-5-neuraminic acid *(Neu5Ac)* having the basic 9-carbon neuraminic acid core modified at the 5-carbon position with an attached acetyl group. Common derivatives of Neu5Ac at the 5-carbon position include: 2-keto-3-deoxy-d-glycero-dgalactonononic acid (KDN) which possesses a hydroxyl group in place of the acetyl group; de-N-acetylation of the 5-N-acetyl group produces neuraminic (Neu); hydroxylation of the S-N-acetyl group produces N-glycolylneuraminic acid *(*Neu5Gc*)*. The hydroxyl groups at positions 4-, 7-, 8- and 9- of these four molecules *(*Neu5Ac, KDN, Neu and Neu5Gc*)* can be further substituted with O-acetyl, O-methyl, O-sulfate and phosphate groups to enlarge this group of compounds. Furthermore, unsaturated and dehydro forms of sialic acids are known to exist.

There is a significant unmet need to develop biological countermeasures to protect warfighters should they be subjected to nerve agents such as sarin and VX in the field. A promising prophylactic is the protein butyrlcholinesterase (BChE), which will bind in a 1:1 stoichiometric fashion to inhibit the action of organophosphosphorous (OP) nerve agents. Natural human BChE (huBChE) derived from plasma has been highly successful at inhibiting OP agents in animal models by maintaining a long circulatory half-life (mean residence time), making it effective for extended periods in the field.

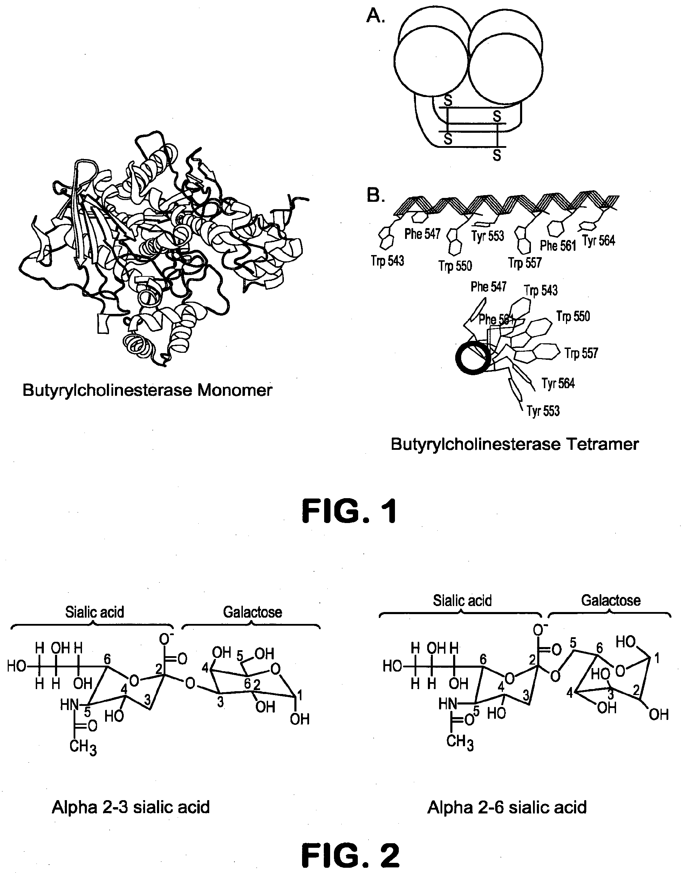

Because the supply of plasma may be limited, alternative sources of the human BChE protein are needed, such as from recombinant expression systems. Human BChE (huBChE) from a recombinant organism represents a potential viable alternative to purification of the enzyme from human plasma. The natural protein demonstrates tetramer assembly and complex glycosylation modifications, therefore expression in a mammalian cell is the best host for this complex protein therapeutic. Specifically, mammalian cells possess the capacity to perform complex post-translational modifications, including the addition and modification of N-glycans (N-linked glycosylation). If processing proceeds completely, N-glycans terminate with a sialic acid (disialylated) although other glycans may terminate in one sialic acid (monosialylated) or galactose if glycosylation processing is not complete. Furthermore, it is now known that there are two different types of sialic acid (Neu5Ac) linkages. One sialic acid linkage involves the linkage of sialic acid in an alpha2-6 configuration to the galactose residue while other sialic acid linkage involves the sialic acid linked alpha2-3 to the galactose residue (Figure 2).

However, recombinant human BChE (rhuBChE) has not been as effective as plasma-derived huBChE, predominantly because the circulatory half life can be many fold shorter resulting in an agent that does not work for long periods in the field. The circulatory half-lives of rhuBChE and human serum BChE have been compared. While the human ChEs reportedly displayed a long mean residence time of about 2,500-3,000 minutes, rhuBChE residence time was nearly than 10 times shorter and in the range of only 50 minutes. This deficiency in MRT has been attributed to both the insufficient sialic acid content as well as the lack of tetrameric assembly as described below.

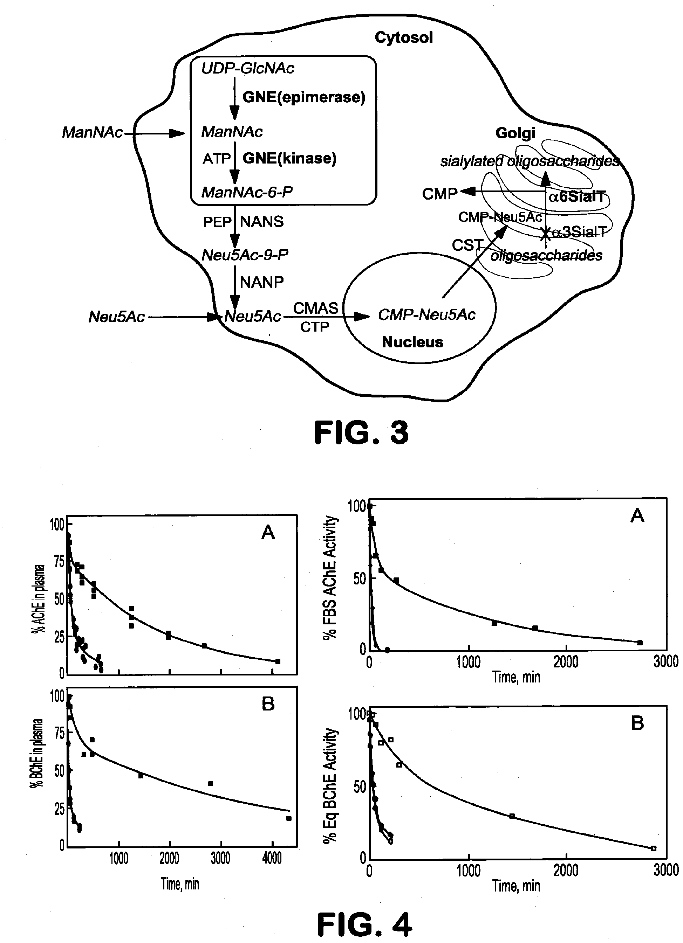

The nature of the shortcomings of current recombinant expression systems for the production of a rhuBChE product that rivals the plasma-derived product may be explained by the following observations. For example, a much more rapid clearance rate for recombinant human acetylcholine esterase (AChE) from the circulation of mice compared to natural AChE from fetal bovine serum (FBS) has been found to be due to the lack of occupied sialic acid sites on the recombinant protein. Furthermore, removal of the sialic acids from the native enzyme decreased the circulatory-half life to just a few minutes as compared to tens of hours for the native protein. The presence of sialic acid on an oligosaccharide can increase a protein's in vivo circulatory half life by prohibiting its binding and removal by the liver asialglycoprotein receptor that removes proteins with glycans terminating in galactose. Hepatic receptors in animals can also remove glycans ending in fucose/*N*-acetylglucosamine or mannose. In order to study the role of sialic acid in multiple native ChEs, mean residence time of natural BChE and AChe from equine sources were examined following intravenous injections in mice before and after the application of neuraminidase (sialidase) treatment to remove the sialic acid residues or glycosidase to remove the entire glycan. Treatment of native BChE and native AChE with neuraminidase or glycosidases lowered the mean residence time (MRT) 10 to 40 fold as shown in Figure 4. The removal of sialic acids lowered the MRT of plasma derived equine BChE from 1437 minutes to 150 minutes. Furthermore, the decline was similar with both the neuraminidase and glycosidase to suggest that sialic acid was playing the key role in maintaining circulatory half life. The findings of the steep decline in MRT for homologous BChE confirm the importance of sialic acid in maintaining the long duration of FBS AChE and Eq BChE in blood.

The reason that current rhuBChE is not effective is because the expression systems used to produce this protein do not have the synthetic capability necessary for generating a product with similar properties as the native huBChE. The two principal limitations of current expression systems are as follows: (1) Sialic acid attachments on glycoproteins are critical for extended circulatory lifetime. However, current expression hosts do not produce enough sialic acids on the rhuBChE glycoprotein and often add alpha2-3 sialic acid linkages, which are different from the alpha2-6 sialic acid linkages that predominate on plasma-derived huBChE. (2) Expression hosts often do not contain sufficient chaperoning capacity to assemble rhuBChE monomers into the proper tetrameric form that also increases circulatory half-life.

Plasma-derived huBChE contains primarily alpha2-6 sialic acid linked to the glycoproteins. Normal CHO cells are unable to generate alpha2-6 linkages because they expression of the alpha2,6-sialyltransferase enzyme is silenced. As a result, recombinant CHO cells generate exclusively alpha2-3 sialic acid. The predominance of the alpha-2,3-linkage in recombinant BChE derived from CHO cells may result in more rapid removal of the sialic acid linkages when the BChE is injected into the body and more rapid removal from the circulatory system. Therefore, it is desirable to engineer CHO cells such that these cells will produce recombinant proteins containing primarily alpha-2,6-linkages. A superior recombinant expression system may be achieved by two-pronged approach. First, CHO cells will be engineered to express alpha-2,6-sialylatransferase and increase overall sialylation. Secondly, CHO cells will be engineered to inhibit expression of the alpha-2,3-sialyltranferase so that these linkages are replaced with alpha2-6 sialic acid linkages. In other words, alpha2-3 sialic acid linkages will be reduced or eliminated contemporaneously with an increase in the alpha2-6 sialic acid content.

In order to more completely understand the extent and quality of glycosylation of serum BChE, a detailed evaluation of N-glycans attached to plasma-derived natural BChE has been undertaken. It has been found that huBChE was highly glycosylated with nine N-glycosylation sites. Analysis of all nine *N*-glycosylation sites revealed that these sites contained principally mono- and di-sialylated *N*-glycans. However, of particular significance was the finding that the sialic acids (Neu5Ac) were predominantly alpha2-6 linked to the galactose, although a few alpha2-3 sialic acid linkages were observed (see Figure 2). In addition, in cases where there was only one sialic acid i.e., mono-sialylated BChE, the linkages were exclusively alpha2-6 linkages. In other words, there were no glycans obtained from plasma-derived huBChE containing only alpha2-3 sialic acid linkages.

As discussed above, CHO cells have been widely used for the production of certain glycoproteins as these hosts are capable of producing post-translationally modified proteins in high yields. These cells will cap some, but not necessarily all, of the galactose residues with sialic acid, which can result in incomplete sialylation of rhuBChE. However, it is also known that the sialic acids added onto recombinant glycoproteins by CHO cells are exclusively alpha2-3 sialic acid linkages. The alpha2-6 sialyltransferase gene in CHO cells is silenced and thus not active in the generation of heterologous proteins such as rhuBChE from CHO. As a result, the widely used Chinese Hamster Ovary production hosts in current usage is incapable of producing rhuBChE with similar alpha2-6 sialic acid linkages that are generated in the native plasma-derived huBChE. In other words, in order to produce a recombinant huBChE that mirrors the endogenous plasma-derived huBChE, it will be impossible to use traditional CHO expression hosts. This presents a particular problem since CHO cells are the most widely used production platform in the biotechnology industry.

Whether a difference in sialic acid processing between the native huBChE (mostly alpha2-6 sialic acid linkages with a few alpha 2-3 sialic acid) and rhuBChE (exclusively alpha 2-3 linkages) from CHO cells will make any difference in the biological properties, including the circulatory residence time, of the resulting product merits exploration. While there has not been a direct comparison, it is worthwhile to examine the relative sensitivity of the two sialic acid linkages. In particular, both sialic acid linkages can be eliminated by sialidases (neuraminidases) that are present in animals. Furthermore, there have been comparisons between the activities of these sialidases. A previous examination of the substrate specificities of sialidases from rat liver and human liver demonstrated more rapid hydrolysis of alpha2-3 sialic acid linkages over the alpha2-6 sialic acid linkages. In short, the alpha2-3 sialic acid linkages may be more susceptible to degradation in animals. Furthermore, this would explain that even though some sialic acid linkages were present in the rhuBChE, the protein possessed a much shorter in vivo circulatory lifetime than plasma huBChE due to the presence of different sialic acid linkages.

One manner to reduce the alpha 2-3 sialic acid content is by lowering the expression of one or more alpha2-3 siafylatransferase genes including ST3gal1, St3gal2, St3gal3, st3gal4, st3gal5, st3gal6 that may be expressed in a CHO or mammalian or eukaryotic cell lines or the activity of the alpha2-3 sialyltransferase activity. The alpha2-3 sialic acid may be reduced using siRNA or other technologies that are used to lower activity level, substrate specificity, or expression of active protein. Specific chemical inhibitors of the protein could also be used. Another alternative is to knock out or otherwise mutate the gene or genes (Stgal1, St3gal2, St3gal3, St3gal4, St3gal5, St3gal6) at the DNA level such as alpha2-3 sialyltransferase activity is reduced or eliminated.

The alpha2-6 sialic acid content can be increased by overexpressing alpha2-6 sialyltransferase (St6gal1, st6gal2). An alternative is to increase the activity of alpha 2-6 sialyltranferase relative to the alpha2-3 sialyltransferase activity by some manipulation. For instance, a genetic modification that results in an increase the alpha2-6 sialyltransferase activity or change the location of the enzyme in the cells so that it is active before the alpha2-3 sialyltransferase can reach the substrate. The substrate specificity may also be altered so that alpha2-6 sialyltransferase encages the CMP-sialic acid substrate before alpha2-3 sialyltransferase can be active. The substrate available for sialylation can be increased by increasing the pools of CMP-sialic acid in the proper compartment through increased production or transport to the site of action of this sialylation substrate.

A cell's endogenous sialidase/neuraminidase activity can be reduced or eliminated so that cells cannot break down the sialic acid once it has been added. This can be achieved by, for example, inhibiting or knocking out a cell's specific neuraminidase activity such as by inhibiting genes for Neu1, Neu2, and or Neu3 using technologies described above for reducing alpha2-3 sialyltransferase activity.

The sialic acid content can be increased by increasing the branching available by overexpression of galactose transferase or otherwise increasing its activity to allow more branches that are available for capping with sialic acid such as alpha2-6. Another approach would be to increase mannosidase and GnT expression or activity levels so that branching can proceed more rapidly.

Increasing the presence of other molecules that may lower the capacity of sialdases in the body to remove or cleave off either alpha2-6 or even alpha2-3 sialic acid. One potential approach would be to increase the expression or activity of a fucosyltransferase that will bind to a sugar group and inhibit neuraminidase activity in the body. Such a fucosyltransferase would be an alpha3fucosyltransferase or (alpha3FucT) encoded by FUT4, FUT5, FUT6, FUT7, FUT8, FUT9 or alpha3,4 fucosyltransferase (FucTLe) FUT3 or alpha2fucosyltransferase (FucTLe) encoded by FUT1, FUT2. Figure 9 shows a modified N-glycosylation pathway of high and low passage LNCaP cells. A main feature of this pathway is the absence of Type I glycans (see Figure 9), implying that glycans characteristic of these cells are type II glycans. The enzyme associated with type II glycans, b4GalT presents increased expression of B4GALT1 and B4GALT3 genes among other genes. The main difference between low and high passage LNCaP cell lines is the increased expression of FucTH in high passage LNCaP cells in both microarray data and mass spectra model predicted enzyme levels.

The circulatory lifetime could also be extended by increasing the length of the branches on N-glycans by increasing the number of polylactosamines by expressing beta3-GlcNAC transferase (iGnT) encoded by B3GNT1, B3GNT2, B3GNT3, B3GNT4. More branches could be added by expressing a branching enzyme β6-GIcNAc-transferase (IGnT) encoded by (GCNT2). These branches then could be sialylated for greater sialic acid content.

Sialic acid attachments on glycoproteins are critical for extended circulatory half-life thus, increasing the overall sialic acid content may be sufficient to prolong circulatory half-life.

A second limitation that causes low retention time in the blood is the absence of tetramer assembly of the BChE protein. While increasing alpha2-6 sialic acid content represents one key component for obtaining a better biological mirror of the natural BChE, another factor that is also important for maintaining extended mean residence time is the proper assembly of the BChE. Plasma-derived BChE is primarily tetrameric in form and in order to obtain a proper biological mimic, it would be desirable to express a recombinant form that is also predominantly tetrameric in form (Figure 8). Indeed, monomeric forms of cholinesterases have exhibited residence times on the order of 40 times shorter than those of tetrameric cholinesterases. In a previous study with huAChE protein, tetramers were found to have a MRT that was more than seven times longer than the residence time of the monomer form. Likewise, the dimer was found to have a residence time that was twice that of the monomer. Interestingly, however, removal of sialic acid from all the forms reduced the residence time of all forms to only five minutes regardless of the assembly state of the protein. These observations imply that multiple removal systems contribute to the elimination of AChE from the circulation.

In the case of tetrameric assembly, size based clearance mechanism may play an important role when monomers and dimers are present instead of tetramers. Unfortunately, rhuBChE produced by unmodified CHO or other mammalian expression systems exhibits a limitation in tetrameric assembly. Indeed, when rhuBChE was expressed in either CHO or HEK cells, the product was generated as a mixture of low residence monomers and dimmers, with less than 10% tetramers present. Thus, the lack of efficient tetrameric assembly in CHO or other cells represents another bottleneck to the efficient production of recombinant BChE with a long circulatory half-life.

CHO cells express primarily monomer forms of the BChE protein and previous studies have shown that the monomer is cleared more rapidly from the body. Thus, it is also desirable to engineer CHO cells with the capacity to increase tetramer assembly. Fortunately, tetramer assembly is facilitated by the presence of the PRAD attachment domain. By co-expressing this PRAD gene along with genes for alpha2-6 sialyltransferase, increased levels of the tetramer protein containing higher levels of 2-6 sialylated glycoproteins will be achieved.

Reasons for the rapid clearance of recombinant BChE from the body include the deficiency of current expression systems, which lack the ability to produce a recombinant product that exhibits the properties critical for long circulatory half-life. The reason for low activity of rhuBChE obtained from current expressions systems are two fold: (1) Sialic acid content is important for a glycoprotein such as BChE to be maintained in the circulation. However, there are not enough sialic acids on the rhuBChE and the type of sialic acid linkage (alpha2-3) is not ideal for long circulatory half-life. In contrast, plasma derived BChE contains mostly alpha2-6 sialic acid linkages on the glycoprotein. (2) BChE is a tetrameric protein but the protein is expressed primarily as a monomer and dimer in recombinant expression systems. Tetramers are maintained in circulation longer than monomers and dimers.

The application of synthetic bioengineering to modify recombinant expression systems by adding the above capabilities will result in the synthesis of a long-lived and active rhuBChE product. The methods described here are transformational in advancing the state-of-the-art by replacing the current inadequate production methods with an expression system that has improved alpha2-6 sialic acid content and enhanced tetramer assembly capabilities. These modifications to the current CHO production host will provide a novel expression system that will enable synthesis of a recombinant huBChE product that is virtually identical in chemical, physical, and bioactivity as the plasma-derived BChE. Furthermore, the rhuBChE will be safer than that sourced from plasma and humans as it will be free from the danger of contamination by adventitious agents present in human donors. In addition, this expression technology will be equally applicable to future OP-scavengers that are glycoproteins like huBChE and must be produced in a form that is long lasting in circulatory system.

Described herein is a method of modifying mammalian cells, such as Chinese Hamster Ovary cells, to synthesize a rhuBChE protein that contains increased 2-6 sialic acid content and higher levels of tetramer assembly. This novel CHO system will be implemented in a GMP production process and the modified rhuBChE will be subsequently tested in animal models in order to demonstrate pharmacokinetics and efficacy similar to the natural huBChE. It is expected that this will result in the development of a commercially viable process for the manufacture of GMP-grade rhuBChE with equivalent physical, chemical and biological properties as the plasma-derived huBChE. Furthermore, this engineered CHO expression system will be applicable to the production of numerous other OP-bioscavengers as they are developed in the coming years.

### EXAMPLE 1

### EXPRESSION OF RECOMBINANT HUMAN BChE (huBChE) IN CHO

The gene for human butyrlcholinesterase (huBChE) will be obtained from a commercial DNA human liver library or other researchers from previous research. As needed, BChE cDNA can be cloned from total liver mRNA. For expression of huBChE in CHO cells, the full length BChE cDNA will be inserted into the pcDNA mammalian expression vector, which also contains a neomycin resistance gene (phuBChE-neo). CHO-K1 cells will be obtained from ATCC and grown up in standard DMEM medium. Then the CHO-K1 cells will be transfected with the phuBChE-neo plasmid using lipofectamine and high level expression clones of huBCHE will be selected using increasing concentrations of G-418. The highest expressing clones will be identified using anti-huBChE antibodies in ELISA assays. From this multiple adherent stable CHO-K1 cell lines expressing monomeric rhuBChE (CHO-rhuBChE) will be obtained.

### EXAMPLE 2

### ENGINEERING RECOMBINANT HUMAN ALPHA2-6 SIALYLTRANSFERASE GENE IN CHO

This example illustrates recombinant expression systems that increase alpha2-6 sialic acid content in glycoproteins by engineering genes for generating alpha2-6 ialyltransferase in CHO.

The first step will be to express the gene for alpha2-6sialyltransferase (ST6GAL1;Pubmed Gene ID: 6480) in CHO-rhuBChE. The gene for human ST6GAL1 will be obtained from a commercial cDNA library. As an alternative, ST6GAL1 cDNA can be cloned from total UNA isolate using reverse transcriptase and human ST6GAL1 gene specific PCR primers. The full length cDNA will be inserted into the pcDNA mammalian expression vector, which also contains a zeocin resistance gene (pST6GAL1-zeocin). Then CHO-rhuBChE cells will be transfected with the pST6GAL1-zeo plasmid using lipofectamin 2000 (Invitrogen) and clonal isolates selected in selection medium containing zeocin antibiotic. This process will afford CHO-rhuBChE-ST6GAL1 clones co-expressing recombinant huBCHE and ST6GAL1, which may be analysed by positive western blot against anti-ST6GAL1 antibody.

### EXAMPLE 3

### INHIBITION OF ALPHA2-3 SIALYLTRANSFERASE

This example illustrates recombinant expression systems that decrease the alpha2-3 sialic acid content in glycoproteins by knockdown or knockout of the alpha2-3sialyltransferase gene.

Sialic acids attached alpha2-3 to recombinant BChE are suspected to be less likely to remain in circulation and more susceptible to sialidases (neuraminidases in the body than alpha2-6 sialic acids. In order to test this hypothesis, the circulatory half-life and structures for cells that a) express alpha2-3 sialic acid (CHO-RhuBChE) b) express both alpha2-3 and alpha2-6 sialic acid (CHO-rhuBChE-ST6GAL1) and c) those that express predominantly alpha2-6 sialic acid attachments (CHO-rhuBChE-ST6GAL1-ST3GAL(-) will be compared. In order to create this third variant, the endogenous Chinese Hamster Ovary (CHO) alpha2-3sialyltransferase gene (St3gal1) will be reduced using siRNA technologies. To select an siRNA sequence to knock down St3gal1 gene, the mRNA sequence for this gene will be entered to the siRNA design tool. The tool will suggest candidate double-stranded siRNA sequences and several St3gal1 siRNAs will be ordered and transfected into CHO-rhuBChE-ST6GAL1 cells to analyze for St3gal1 gene knockdown efficiency. The siRNA sequence which provides the most efficient St3gal1 gene knockdown will be synthesized and ligated into pSilencer™ 4.1-CMV-puro siRNA expression vector. The resulting pSilencer™ 4.1-CMV- ST3Gal1(-)shRNA-puro plasmid will be transfected into CHO-rhuBChE-ST6GAL1 using lipofectamine and clonal isolates selected with puromycin antibiotic. As an alternative, the use of zinc fingers as a method for completely knocking out the St3gal1 will be employed. A pair of zinc finger nucleases will be designed to generate a double strand DNA break within the St3gal1 target site which will lead to a permanent mutation. Zinc finger nucleases will be transfected into CHO-rhuBChE-ST6GAL1 cells. After transfection dilution cloning (one cell per well) will be performed to isolate the single clones from transfected CHO-rhuBChE-ST6GAL1-St3Gal1(-) cell pool. The single cell derived colonies will be then analyzed for St3gal1 gene disruption using PCR analysis. This method will afford multiple CHO-rhuBChE-ST6GAL1-ST3Gal1(-) clones expressing recombinant huBCHE and STGAL1 with reduced or knocked out ST3Gal1 expression. Reduced alpha2-3 sialic acid levels for CHO-rhuBChE-STGAL1-St3gal1(-) will be observed using alpha2-3 neuraminidase treatment and lectins specific 2-3 sialic acid linkages.

### EXAMPLE 4

### TETRAMERIC ASSEMBLY OF RECOMBINANT HUMAN BChE IN CHO

This example illustrates that glycoprotein tetramers can be obtained from recombinant expression systems by co-expressing the PRAD of ColQ gene.

The lack of a long circulatory half life of recombinant huBChE is due at least in part (along with sialic acid deficiency) to the inability of recombinant expression host to produce tetramers. It has been shown that the carboxy domain of the BChE monomers interacts with the proline-rich attachment domain (PRAD), a 17-residue peptide, of the Colq gene. This domain is critical in facilitating the assembly of BChE into tetramers. Consequently the PRAD of Colq gene obtained from a commercial cDNA library or previous researchers will be cloned into a pcDNA mammalian expression vector carrying a hygromycin resistance genes (pPRAD-hygro). As an alternative, the cDNA coding for the 17-residue peptide can be synthesized chemically. Next the cell lines developed in the preceding examples (CHO-rhuBChE, CHO-rhuBChE-STGAL1 and CHO-rhuBChE-ST6GAL1-ST3Gal1(-)) will be transfected with the pPRAD-hygro in the presence of lipofectamine and selected in hygromycin-containing medium order to incorporate the PRAD chaperone for tetramer assembly. Incorporation of pPRAD-hygro may precede incorporation of ST6GAL1 or knockdown of STGal1(-). The processes described herein are interchangeable and do not need to be performed in any particular order because all the genes have separate antibiotic resistances and all will be incorporated into CHO. Clones expressing the PRAD chaperone for tetramer assembly will be selected in hygromycin and the highest expressing clones will be identified using anti-PRAD antibodies. From this multiple stable PRAD expressing CHO cell lines will be obtained including CHO-rhuBChE-PRAD, CHO-rhuBChE-STGAL1-PRAD and CHO-rhuBChE-ST6GAL1-ST3Gal1(-)-PRAD. This method will yield clones of CHO-rhuBChE-PRAD and CHO-rhuBChE-STGAL1-ST3Gal1(-), which may be analyzed by a positive screening of PRAD expression on western blot and an increase in percentage of tetramers (preferably above 50%) produced by CHO cell lines expressing PRAD.

### EXAMPLE 5

### ANALYSIS OF TETRAMERIC ASSEMBLY & SIALIC ACID CONTENT OF RECOMBINANT HUMAN BChE IN CHO

This example illustrates the qualification and quantification of sialic acid linkages and tetrameric glycoproteins obtained by the above recombinant expression systems, in particular compared to natural human plasma BChE.

A tetramer assay will be developed and tetramers in plasma derived BChE and rhuBChE from unmodified and modified CHO will be compared. The extended circulatory half-life of the natural human form of huBChE is due at least in part to the presence of predominantly tetramers in the plasma derived product. In order to monitor and compare the characteristics of native and recombinant BChE, multiple assays to monitor the assembly state of BChE will be contemplated. It will also be important to determine if the expression of heterologous PRAD increases the percentage of tetramers generated by recombinant CHO cells. The level of tetramer versus monomer can be compared using sucrose gradient ultracentrifugation, PAGE, or size exclusion chromatography. For sucrose gradient centrifuguation, huBChE is applied to a linear 5-20% linear sucrose gradients and centrifuged at 30,000g for 18 hours in an ultracentrifuge. Gradients are fractionated and assayed for BChE activity. The tetramers will sediment to a much lower sucrose density than is observed for the monomers and dimers. An alternative quantitative method for measuring the amount of tetramers is through the use of size exclusion chromatography (SEC) (PNAS). The BChE protein is run on an HPLC system containing a SEC KW-803 column from Shodex which includes an exclusion limit of 1.7 x 10⁵ and 21,000 theoretical plates. Samples are detected using a UV detector and fractions are collected in aliquots followed by analysis for BChE activity using the standard Ellman assay. Data is then plotted as BChE activity versus collection interval in which the tetramers will emerge from the column first followed by dimers and finally monomers. By using area counts, the percentages of tetramer, dimer, and monomer can be determined. Thus, a quantitative method that evaluates the percentage of tetramers, dimers, and monomers of BChE from human plasma and unmodified and engineered CHO cells is afforded. This assay can be used to demonstrate natural human derived plasma contains greater than 70% tetramer and recombinant tetramer levels increase follow PRAD expression.

### EXAMPLE 6

### ANALYSIS OF 2-6 AND 2-3 SIALIC ACID CONTENT AND GLYCAN COMPOSITION OF PLASMA DERIVED & RECOMBINANT HUMAN BChE

This example describes a method to quantitatively measure the percentage of the alpha2-3 and alpha2-6 sialic acid content and the complete glycan structures of plasma derived and recombinant huBChE.

Another reason for the extended circulatory half-life of the natural plasma form of huBChE is the presence of extensive alpha2-6 sialic acid on the BChE that prevents by receptors in the liver and other organs from removing proteins that contain non-sialylated structures. It is desirable, therefore, to increase the alpha2-6 linkages and decrease the alpha2-3 linkages on rhuBChE. The 2-6 and 2-3 sialic acid content on native plasma BChE will be examined and the determined level will be compared to the sialic acid content of rhuBChE obtained from the wild-type and engineered CHO cell lines. First, the total sialic acid content will be measured and quantified using lectin microarrays specific for sialic acid. If the sialic acid content differs, then a differential binding pattern will be observed for the recombinant and plasma-derived forms. Next the protein will be treated with an alpha2-3 specific neuraminidase (sialidase) in order to remove these specific sialic acids and then the sialic acid content will subsequently be quantified again using lectin microarrays. As an alternative HPLC analysis can be used to quantify the amounts of sialic acid glycans following alpha2-3 neuraminidase treatment. Finally, to generate complete glycan structural details, complementary mass spectrometry (MS) analysis will be performed using MALDI-AXIMA resonance mass spectrometer on the glycans released from isolated glycoprotein as shown in Figure 11. This state of the art method for characterizing glycan structures uses MS profiling that is typically coupled with liquid chromatography (LC) to separate complex glycan mixtures. The use of combined HPLC-MALDI analysis methods has proven to be successful for detecting N-linked glycopeptides and glycans. The spectra acquisition of a MALDI-AXIMA resonance mass spectrometer will make possible identifying detailed glycan structures that may not be detectable by other MS units. This LC -MS/MSn analysis of glycans will generate a collection of molecular weights in multiple dimensions that are representative of the N-glycan profile for huBChE from human plasma, normal CHO, and CHO engineered with different sialyltransferases. That natural human derived plasma contains significant sialic content and the alpha2-6 sialic acid content of rhuBChE increases following CHO cell engineering will be demonstrated.

### EXAMPLE 7

### OPTIMIZATION OF rhuBChE PRODUCTION PROCESS

This example illustrates a protocol for obtaining suspension clonal cell lines of CHO-rhuBChE, CHO-rhuBCHE-ST6GAL, CHO-rhuBCHE-ST6GAL-ST3GAL(-), and CHO-rhuBCHE-ST6GAL-ST3GAL(-)-PRAD in serum-free medium.

In order to produce significant amounts of recombinant huBChE for animal trials and clinical trials in the future, a process must be developed that is appropriate to GMP manufacturing. Therefore it will be important to identify CHO clones which can grow robustly and are amenable to scale-up as new cell lines are developed. In order to make a cell culture process that is scale-able, the cells must be adapted to suspension culture while still producing desirable yields of rhuBChE. Secondly, and equally important, will be the elimination of serum from the culture medium as the presence of serum complicates the capacity to purify secreted rhuBChE from the CHO cell culture. The procedures will be described for CHO-rhuBChE but similar methods will be applied for CHO-rhuBCHE-ST6GAL, CHO-rhuBCHE-ST6GAL-ST3GAL(-), and CHO-rhuBCHE-ST6GAL-ST3GAL(-)-PRAD. First, multiple attachment-dependent clones expressing rhuBChE will be progressively weaned off serum through repeated passaging in progressively lower concentrations of serum in combination with increasing percentages of CHO commercial serum free medium. Cell robustness will be monitored in suspension cultures using shaker flasks or spinner flasks and by measuring growth rates and maximum viable cell densities over time for approximately 5 to 10 clones from each successful transfection. The production rate of BChE of each clone will also be monitored using the activity measurements in order to determine which clone provides the highest yields. For the most robust clones elucidated, the levels of tetramer assembly and sialic acid content will also be evaluated in order to ensure generation of favorable product profiles. In all cases, the cells will only be exposed to registered components so that the cell lines can eventually be converted into a GMP facility for production of BChE for animal and future clinical trials. From these studies, suspension cell lines will be obtained that can grow to high cell densities in serum free culture and produce recombinant huBChE in quantities sufficient for animal trials. It is possible to achieve doubling times of less than 24 hours for each clone with suspension cell densities greater than 1 x 10⁶ cells/mL with production of rhuBChE at levels of 1 unit/mL or higher.

### EXAMPLE 8

### PURIFCATION PROTOCOLS FOR RECOMBINANT AND NATURAL PLASMA-DERIVED BChE

This example describes a protocol for the purification of tetramers and monomers of rhuBChE.

In order to obtain sufficient rhuBChE and natural BChE for animal trials and chemical/physical analysis, it will be essential to purify the recombinant protein from cell culture supernatants and the natural protein from plasma. For the recombinant protein, the cell culture supernatant will first be separated from the cells by centrifugation. Next, the recombinant protein supernatant or diluted plasma will be loaded onto a procainamide-Sepharose chromatography column. Following loading, the columns will be washed with 25 mM sodium phosphate buffer and then eluted with a linear gradient of 0.05-1.0 M NaCl for monomelic and tetrameric BChE forms. Fraction elution is determined by measuring Absorbance at 280 nm and then separate fractions are collected and monitored for BChE activity. Those fractions containing BChE will be pooled, concentrated, and desalted by ultrafiltration. This approach can purify native BChE to 40 to 50% and the recombinant BChE to nearly 70%, In order to increase the purity of the fractions containing BChE, an ion exchange column can be added to the process. This protocol can furnish natural human derived plasma BChE at a purity of 50% and recombinant huBChE at purity above 70%.

### EXAMPLE 9

### SCALE-UP CHO CELL CULTURE PROTOCOL FOR GLP PROCESS

This example illustrated processes for the different CHO clones obtained from the recombinant expression systems described above.

The scale-up CHO cell culture protocol for GLP process is as follows. Techniques will be developed which take small scale shaker and spinner cultures and scale them up to a process to provide sufficient rhuBChE for animal trials in mice and analytical measurements. The amount of sample needed for animal trials is approximately 100 units/mouse. If we assume a rhuBChE production rate of 1 unit/mL, then approximately 600 units will be required for a recombinant huBChE test on 6 mice. Assuming a 50% purification rate, then 1200 mL will be required for mice trials. In order to make sufficient additional protein for glycan and tetramer analysis, approximately 2.5 liters of culture will be produced for each cell line and trial in a GLP Process. For the cell culture process, wave bioreactors will be applied that can be easily incorporated into most GMP facilities. As an alternative culture platform, computer-controlled bioreactors will also be contemplated. Multiple master and working cell banks that can be used at GLP and GMP levels will be generated from the optimal producing clones. Initially, CHO-rhuBChE clone from a working bank will be grown up to 200 mL in a shake flask. The media and cell seeding parameters will be varied in the wave bioreactor or cell culture bioreactor in order to optimize the final cell densities of CHO cells and the final concentration of BChE in the culture medium. A number of bioreactor parameters will be followed including glucose, oxygen, pH, and glutamine and fed batch addition of nutrients; these will be evaulated in order to maximize cell densities for the scaled up process. 2.5 liter scale-up processes will be established for each of the following CHO cell clone: 1) CHO-rhuBChE; 2) CHO-rhuBCHE-ST6GAL, 3) CHO-rhuBCHE-ST6GAL-ST3GAL(-), and 4) CHO-rhuBCHE-ST6GAL-ST3GAL(-)-PRAD. This process will result in production of at least 1 unit/ml of rhuBChE in each 2.5 liter process with cell densities at or above 1 x 10⁶ cells/mL in suspension culture.

### EXAMPLE 10

### CHO CELL CULTURE MANUFACTURING OF rhuBChE UNDER GMP CONDITIONS

This example illustrates processes amenable for GMP manufacture of rhuBChE obtained from the recombinant expression systems described above.

A procedure for CHO cell culture manufacturing of rhuBChE amenable to GMP manufacturing and animal trials is as follows. A 2.5 liter process amenable to GMP manufacturing will be implemented in order to culture and purify at least 600 units each of purified rhuBChE from the following four cell lines: 1) CHO-rhuBChE; 2) CHO-rhuBCHE-ST6GAL, 3) CHO-rhuBCHE-ST6GAL-ST3GAL(-), and 4) CHO-rhuBCHE-ST6GAL-ST3GAL(-)-PRAD. This amount of protein represents approximately 0.9 mg each of purified rhuBChE protein. An additional mg of rhuBChE protein will be purified from each sample for tetramer assembly analysis and analysis of sialic acid content. These production studies studies will be performed at the Cell Processing and Gene Therapy (CPGT) core at the Johns Hopkins Kimmel Cancer Center. This facility employs Good Manufacturing Practices (cGMP) appropriate for animal, phase I, and phase II studies. The facility, constructed with FDA input and validated in 2000, includes an 1800 ft² cGMP manufacturing facility containing four independent, HEPA filtered, class 10,000 manufacturing suites, and a 400 ft² Process Optimization Laboratory (POL). The POL is responsible for transitioning manufacturing processes based on research laboratory technologies to cGMP compliant productions. Hence, the GMP feasibility studies for this proposal will be carried out in the POL. The POL includes a restricted access laboratory and is equipped with three Biological Safety Cabinets, 2 controlled rate freezers (Planer Kryo and Forma Cryomed) two Stericult incubators, a COBE 2991 cell washer, two low speed centrifuges, a -80 °C freezer, a microscope, a 2-8 °C refrigerator, a balance and a water bath. Wave bioreactors and cell culture bioreactors will be incorporated in order to facilitate GMP-amenable manufacturing at the 2.5 liter scale of the current study. All equipment is quality controlled with a preventative maintenance plan and schedule so that cell based processes optimized by the POL can be exactly implemented in the GMP suites. Development study reports prepared by the POL manager can be used by investigators to support product specifications described in regulatory submissions and IND/IDE CMC. Implementation of the processes described above into the POL facility is planned in order to rapidly achieve a GMP-amenable manufacturing process. In short, a clone will be taken from the working scale bank, grown up in a shaker flask, and then transferred to a 2.5 liter Wave bioreactor or alternative bioreactor configuration that is optimized growth, Purification will be performed using centrifugation followed isolation on an FPLC column. This process will result in production of at least 1 unit/ml of rhuBChE in each 2.5 liter process with cell densities at or above 1 x 106 cells/mL in suspension culture.

### EXAMPLE 11

### PHARMACOKINETIC AND PHARMACODYNAMIC STUDIES OF RECOMBINANT HUMAN BChE

This example illustrates a pharmacokinetic and pharmacodynamic comparison of plasma-derived huBChE to rhuBChE obtained from unmodified and engineered CHO cells in mice.

Pharmacokinetic and pharmacodynamic studies in mice will be performed as follows. All animal studies under consideration will be reviewed and approved by the animal care and use core facility at Johns Hopkins University prior to implementation. In order to demonstrate that the engineered CHO cells cultured and processed in a GMP amenable environment produce rhuBChE that closely mirrors the circulatory stability of plasma-derived BChE, its pharmacokinetic and pharmacodynamic properties in Balb/c mice will first be determined. Four groups of 6-8-week-old BALB/c mice (*n* = 6 per group), will be injected i.m. with 100 U of either (1) native plasma-derived huBChE, (2) rhuBChE from unmodified CHO (CHO-rhuBChE) cells, (3) rhuBChE from CHO-rhuBCHE-ST6GAL-ST3GAL(-)-PRAD or (4) saline (negative controls). The pharmacokinetics of rhuBChE from (5) CHO-rhuBCHE-ST6GAL and (6) CHO-rhuBCHE-ST6GAL-ST3GAL(-) will also be analyzed in order to determine which of the three factors (tetramer assembly [PRAD], 2-6 sialic acid addition [ST6GAL], or replacement of 2-6 sialic acid with 2-3 sialic acid linkages [ST6GAL(+)-ST3GAL(-)] is most critical for reducing clearance rates. Prior to and at 1, 2, 4, 6, 8, 24, 48 and 96 hours post-injection, 5 µl of blood will be taken from the tail vein, diluted in 95 µl of water and assayed for BChE activity using 1 mM butyrylthiocholine (BTC) and 0.5 mM 5,5'-dithiobis 2-nitrobenzoic acid (DTNB) in 50 mM sodium phosphate buffer pH 8.0 at 22 °C. The formation of product will be followed by measuring the increase in absorbance of 5-thio-2-nitrobenzoic acid at 412 nm using a molar extinction coefficient of 13,600 M⁻¹. Activity will be reported as U/ml, where 1 U represents 1 µmole of BTC hydrolyzed per min (Ellman assay). Four pharmacokinetic parameters, based on the time course of BChE clearance in blood will be calculated using a computational program for non-compartmentalized analysis: Mean residence time (MRT), peak plasma BChE activity (Cₘₐₓ), terminal half-life *(T*_{1/2}*)* and area under the curve (AUC). This assay measures activity (pharmacodynamics) of the BChE rather absolute level of the drug. In order to convert the catalytic activity to the absolute protein level (associated with pharmacokinetics) for rhuBChE and plasma-derived huBChE, the specific activity per mg BChE protein can be determined. The activity is measured using the Ellman assay above and the protein content of the purified protein is obtained from A280 using an extinction coefficient of 1.88 for a 1 mg/ml solution. The values of pharmacokinetic parameters for plasma-derived BChE with those obtained for unmodified CHO-rhuBChE and the CHO-rhuBChE-STGAL1-ST3GAL(-)-PRAD will be compared. If cell engineering efforts are successful, the pharmacokinetic parameters of rhuBChE from the modified CHO cell lines described above will be comparable to those for plasma-derived values and superior to the other CHO cell lines (unmodified and those missing one or more of these modifications). In the event that the AUC and MRT are lower for the modified CHO cell line-derived rhuBChE, the analytical comparison of glycan structure and tetramer assembly will dictate a strategy for making the engineered variant even more similar to native huBChE. This protocol will enable determination of AUC and/or MRT for product derived from engineered cells at least 50% higher than that for the unmodified product, and AUC and/or MRT at least 75% of that for plasma derived human BChE.

### EXAMPLE 12

### EFFICACY STUDIES OF RECOMBINANT HUMAN BChE

This example illustrates *in vitro* efficacy comparison of BChE in blood samples from mice injected with plasma-derived huBChE or rhuBChE expressed in both unmodified and engineered (with sialic acid and tetramer assembly).

Efficacy studies will be performed as follows. To examine whether the rhuBChE is as efficacious as the form derived from plasma in scavenging nerve agents, *in vitro* inhibition studies will be performed using nerve agent analogs. The principal analogs that will be used will be diisopropyl fluorophosphate (DFP), C₆H₁₄FO₃P and MEPQ. Blood samples will be withdrawn from mice injected with rhuBChE or plasma-derived huBChE, and incubated with various amounts of OP analogs for 2 hours at 25 °C. Residual enzyme activity will be assayed by the standard Ellman assay. The residual enzyme concentration will be plotted against the number of equivalents of OP agent in solution. A comparison between the *in vitro* activity of the rhuBChE from normal CHO and engineered CHO to the plasma derived huBChE will indicate if the engineered CHO cells are generating a form of the huBChE that is as efficacious as the plasma-derived form. Similar *in vitro* studies could also be performed using OP agents such as soman or VX. This protocol will enable detection of positive efficacy values for rhuBChE from engineered CHO cells that are at least 75% of the values from plasma-derived huBChE.

### EXAMPLE 13

### IMMUNOGENICITY STUDIES OF RECOMBINANT HUMAN BChE

This example illustrates a comparison of immunogenicity of huBChE with rhuBChE obtained from both unmodified and engineered (with sialic acid and tetramer assembly) CHO cells.

Immunogenicity studies will be performed as follows. To examine the immunogenicity of rhuBChE and plasma-derived huBChE, blood samples drawn from mice injected with these enzymes will be analyzed for antibody responses by ELISA assays using anti-huBChE antibodies, Groups of mice (n=6 per group) will be subjected to one or two injections (spaced by four weeks) of the huBChE from each of the three sources: 1) plasma, 2) unmodified CHO and 3) engineered CHO. The presence of circulating anti-huBChE antibodies in mouse blood will be determined by (ELISA). Briefly, 96-well plate will be coated with 50 µl of the huBChE solution (0.2 U/well) in phosphate-buffered saline (PBS). After washing, 50 µl each of 5-fold serial dilutions (ranging from 1:200 to 1:125,000) of mouse blood will be added and incubated overnight at. The ELISA activity will then be determined by detection using a (HRP)-conjugated goat anti-mouse IgG. The absorbance will be measured at 405 nm, and antibody concentrations will be calculated from standard curves. The samples will be examined against all three formulations of BChE in order to consider specific antibody responses against sialic acid or various forms of assembled BChE. A comparison of the activity levels will indicate which, if any, form of the BChE elicits the greatest immune response in mice. Anti-huBChE antibody titers for rhuBChE from engineered CHO cells that are similar to the anti-huBChE antibody titers elicited by plasma derived human BChE will be afforded. In the event of expression of a rhuBChE that displays pharmacokinetic efficacy properties similar to those of plasma-derived huBChE, the pharmacokinetics of this enzyme in a non-human primate model will subsequently be characterized.

## Claims

1. An isolated mammalian cell comprising a heterologous alpha2-6 sialyltransferase nucleic acid sequence, optionally wherein the cell is a CHO cell; further comprising a nucleic acid sequence that decreases expression of or silences an alpha2-3sialyltransferase gene; or further comprising means for knock-out of the alpha2-3sialyltransferase gene.

2. The cell according to claim 1 wherein the nucleic acid sequence that decreases expression of or silences the alpha2-3sialyltransferase gene or the means for knock-out of the alpha2-3sialyltransferase gene is selected from the group consisting of antisense, siRNA, miRNA and a zinc finger nuclease.

3. The cell according to claim 1 or 2, wherein the alpha2-3sialytransferase gene is selected from the St3gal1 St3gal2, St3gal3, St3gal4, St3gal5, St3gal6 gene and a combination thereof.

4. A method for the biosynthesis of an alpha2-6-rich glycoprotein comprising culturing a cell of claim 1 under conditions to co-express a nucleic acid sequence that encodes a peptide or protein, further comprising inhibiting expression of alpha2-3 sialyltransferase.

## Patentansprüche

1. Isolierte Säugerzelle, umfassend eine heterologe Alpha2-6-sialyltransferasenukleinsäuresequenz, wobei es sich bei der Zelle gegebenenfalls um eine CHO-Zelle handelt; weiterhin umfassend eine Nukleinsäuresequenz, die die Expression eines Alpha-2,3-sialyltransferasegens vermindert oder dieses ausschaltet; oder weiterhin umfassend Mittel für das Knock-Out des Alpha-2,3-sialyltransferasegens.

2. Zelle nach Anspruch 1, wobei die Nukleinsäuresequenz, die die Expression des Alpha-2,3-sialyltransferasegens vermindert oder dieses ausschaltet oder das Mittel für das Knock-Out des Alpha-2,3-sialyltransferasegens aus der Gruppe bestehend aus Antisense, siRNA, miRNA und einer Zinkfingernuklease ausgewählt ist.

3. Zelle nach Anspruch 1 oder 2, wobei das Alpha-2,3-sialyltransferasegen aus dem St3gal1-, St3gal2-, St3gal3-, St3gal4-, St3gal5-, St3gal6-Gen und einer Kombination davon ausgewählt ist.

4. Verfahren zur Biosynthese eines alpha2-6-reichen Glykoproteins, umfassend das Kultivieren einer Zelle nach Anspruch 1 unter Bedingungen zum Koexprimieren einer Nukleinsäuresequenz, die für ein Peptid oder Protein codiert, weiterhin umfassend das Hemmen der Expression von Alpha-2,3-sialyltransferase.

## Revendications

1. Cellule de mammifère isolée comprenant une séquence d'acides nucléiques d'une alpha2-6 sialyltransférase hétérologue, la cellule étant en option une cellule CHO ; comprenant en outre une séquence d'acides nucléiques qui diminue l'expression d'un gène d'une alpha-2,3-sialyltransférase ou qui le rend silencieux ; ou comprenant en outre un moyen pour l'inactivation du gène d'une alpha-2,3-sialyltransférase.

2. Cellule selon la revendication 1, dans laquelle la séquence d'acides nucléiques qui diminue l'expression du gène d'une alpha-2,3-sialyltransférase, ou qui le rend silencieux, ou le moyen pour l'inactivation du gène d'une alpha-2,3-sialyltransférase est choisi dans le groupe constitué par un antisens, un ARNsi, un miARN et une nucléase à doigt de zinc.

3. Cellule selon les revendications 1 ou 2, dans laquelle le gène d'une alpha-2,3-sialyltransférase est choisi parmi les gènes St3gal1, St3gal2, St3gal3, St3gal4, St3gal5, St3gal6 ainsi qu'une combinaison de ceux-ci.

4. Procédé destiné à la biosynthèse d'une glycoprotéine riche en alpha2-6, comprenant la culture d'une cellule, selon la revendication 1, dans des conditions permettant d'exprimer conjointement une séquence d'acides nucléiques qui code pour un peptide ou une protéine, comprenant en outre l'inhibition de l'expression d'alpha-2,3-sialyltransférase.
